(19) Europäisches Patentamt
European Patent Office
Office européen des brevets

(11) **EP 2 666 429 A1**

(12) **EUROPEAN PATENT APPLICATION**
published in accordance with Art. 153(4) EPC

(43) Date of publication:
**27.11.2013 Bulletin 2013/48**

(51) Int Cl.:
***A61B 19/00*** *(2006.01)*  ***A61B 17/28*** *(2006.01)*
***B25J 15/08*** *(2006.01)*

(21) Application number: **12758029.8**

(22) Date of filing: **09.03.2012**

(86) International application number:
**PCT/JP2012/056156**

(87) International publication number:
**WO 2012/124635 (20.09.2012 Gazette 2012/38)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **11.03.2011 JP 2011054430**

(71) Applicant: **Olympus Corporation
Shibuya-ku
Tokyo 151-0072 (JP)**

(72) Inventors:
• HYODO Ryoji
  **Tokyo 151-0072 (JP)**
• KISHI Kosuke
  **Tokyo 151-0072 (JP)**

(74) Representative: **Schicker, Silvia
Wuesthoff & Wuesthoff
Patent- und Rechtsanwälte
Schweigerstrasse 2
81541 München (DE)**

(54) **MEDICAL TREATMENT TOOL AND MANIPULATOR**

(57)    This medical treatment tool (1) includes: an end effector (10) which has a first forceps piece (11) and a second forceps piece (12) of which at least one is supported so that it is capable of being rotated relative to a cover member; an operating member (20) which is configured to advance or retract in its own axial direction relative to the cover member so that the pair of forceps pieces rotates and opens/closes; and a link member (15, 17) whose distal end portion (15A, 17A) is coupled to a rotatable forceps piece, and whose proximal end portion (15B, 17B) is coupled to the operating member; wherein a drive axis of the proximal end portion (15B, 17B) is parallel with an axis of the operating member (20); the distance between the distal end portion (15A, 17A) and the drive axis of the proximal end portion (15B, 17B) is shorter than the length of the link member; and the length (L1) of projection on the drive axis of a line segment which connects the second end portion and a center of rotation of the pair of the first forceps piece (11) and the second forceps piece (12) is shorter than the length (L2) of projection on the drive axis of a line segment which connects the distal end portion (15A, 17A) and the center of rotation.

FIG. 3

# Description

[Technical Field]

The present invention relates to a medical treatment tool, and to a manipulator provided therewith. Priority is claimed on Japanese Patent Application No. 2011-054430, filed March 11, 2011, the content of which is incorporated herein by reference.

[Background Art]

Conventionally, in the medical field, medical treatment tools are used for conducting procedures by grasping biological tissue or surgical implements and the like. These medical treatment tools are, for example, attached to manipulators which configure a medical manipulator system of a master-slave system or, for example, are inserted through a forceps channel of an endoscope, and are introduced into a body cavity of a patient or the like for use in various procedures.

As one example of a medical treatment tool, Patent Document 1 discloses a grasping forceps which is provided with a forceps portion which can be opened and closed. A wire is connected to the forceps portion via a link mechanism. The wire is inserted through a coil sheath. The forceps portion is opened or closed when this wire is pushed or pulled to advance or retract it in a longitudinal direction.

With respect to the grasping forceps described above, there is a need to further strengthen the grasping force of the forceps portion so that implements such as suture needles or tissue and the like can be firmly grasped. In order to meet this need, a manipulator which is provided with a so-called toggle mechanism (servomechanism, force enhancement mechanism) is offered in Patent Document 2.

[Prior Art Documents]

[Patent Document]

[Patent Document 1] Japanese Examined Utility Model Application, Second Publication No. H6-1696
[Patent Document 2] Japanese unexamined patent application, First publication No. 2007-301692

[Summary of the Invention]

[Problems to be Solved by the Invention]

However, in the manipulator disclosed in the Patent Document 2, a link which constitutes of the toggle mechanism is coupled with a long round hole and a slidable pin. Then, the toggle mechanism actuates by sliding the slidable pin into the long round hole. Here, due to a friction between the slidable pin and the long round hole, there is a problem that the forceps portion does not move smoothly, or the transmission efficiency of the operation input decreases.

The present invention has been made in view of the above matter, and an object of the present invention is to provide a medical treatment tool and a manipulator which are able to efficiently transmit a manipulation input and to generate a large gripping force with a small manipulation input.

[Means for Solving the Problem]

According to a first aspect of the present invention, a medical treatment tool includes: an end effector which has a pair of jaw members of which at least one is supported so that it is capable of being rotated relative to a base; an operating member which is configured to advance or retract in its own axial direction relative to the base so that the pair of jaw members rotates and opens/closes; and a link member of which a first end portion of a link member is rotatably coupled to the jaw member, and a second end portion of the link member is coupled to the operating member. The drive axis of the second end portion is parallel with the axis of the operating member; the distance between the first end portion and the drive axis of the second end portion is shorter than the length of the link member; and the length of projection of a line segment which connects the second end portion and the center of rotation of the pair of jaw members on the drive axis is shorter than the length of projection of a line segment which connects to the first end portion and the center of rotation on the drive axis.

According to a second aspect of the present invention, in the medical treatment tool, the first end portion of the link member is connected to the operating member on the operating member side from the center of rotation of the pair of jaw members.

According to a third aspect of the present invention, the medical treatment tool further includes a stopper being configured to prevent the length of projection of a line segment which connects the second end portion and the center of rotation on the drive axis from being equal to or more than the length of projection of a line segment which connects the first end portion and the center of rotation on the drive axis when the operating member is moved.

According to a fourth aspect of the present invention, in the medical treatment tool, the stopper is an elongate hole which is formed in the base and which extends in the direction of movement of the operating member, and a connecting shaft which connects the second end portion and the operating member is inserted into the elongate hole.

According to a fifth aspect of the present invention, the manipulator is equipped with the medical treatment tool of the first to the fourth aspects of the medical treatment tool.

[Effects of the Invention]

According to the medical treatment tool and the manipulator of the invention, it is possible to efficiently transmit a manipulation input and to generate a large gripping force with a small manipulation input.

[Brief Description of the Drawings]

Fig. 1 is a diagram which shows one example of the configuration of a medical manipulator system that applies the medical treatment tool of an embodiment of the present invention.
Fig. 2 is a diagram which shows the distal end side of the medical treatment tool of a first embodiment of the present invention.
Fig. 3 is a diagram which shows the interior of the distal end side of the medical treatment tool of the first embodiment of the present invention.
Fig. 4 is a diagram which shows a condition in which the end effector of the medical treatment tool of the first embodiment of the present invention opens.
Fig. 5 is a schematic view which serves to explain a mechanism of grasping force intensification.
Fig. 6 is a diagram which shows the distal end side of the medical treatment tool of a second embodiment of the present invention.
Fig. 7 is a diagram which shows a condition in which the end effector of the medical treatment tool of the second embodiment of the present invention opens.
Fig. 8 is a diagram which shows the interior of the distal end side of a medical treatment tool of a third embodiment of the present invention.
Fig. 9A is a diagram which shows the distal end side of a medical treatment tool of a fourth embodiment of the present invention.
Fig. 9B is a diagram which shows the distal end side of a medical treatment tool of the fourth embodiment of the present invention.

[Description of Embodiments]

A first embodiment of the present invention is described below, but, before that, an example of a medical manipulator system to which the medical treatment tool (hereinafter simply referred to as "treatment tool") and manipulator of the present embodiment are applied.

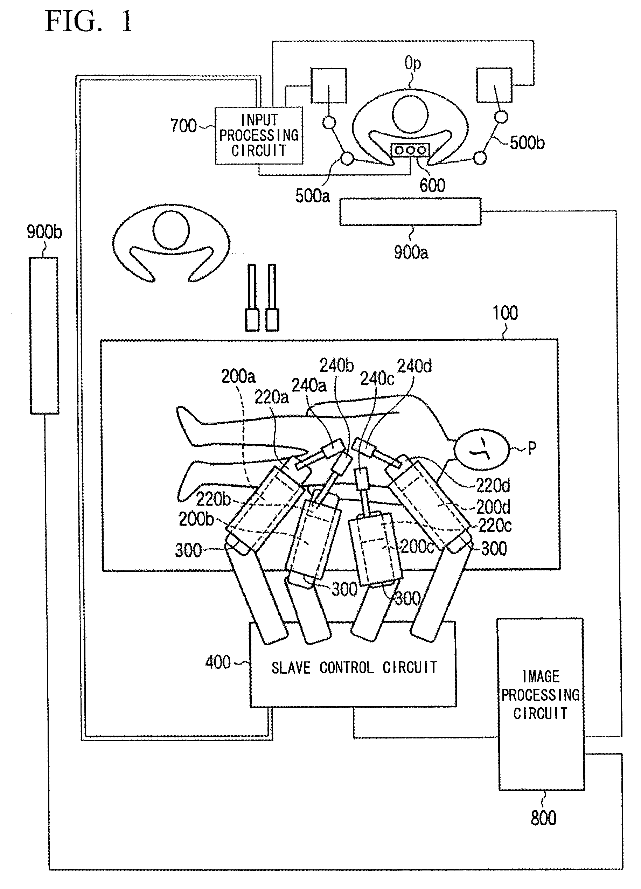

Fig. 1 is a diagram which shows one example of a medical manipulator system; it shows a medical manipulator system with a master-slave system. A medical manipulator system with a master-slave system has two types of arms, which are master arms and slave arms. A medical manipulator system with a master-slave system is a system which conducts remote control of slave arms so that they follow the actions of a master arm. The manipulator of the present embodiment may be applied as such a slave arm.
The medical manipulator system shown in Fig. 1 has an operating table 100, slave arms (manipulators) 200a-200d, a slave control circuit 400, master arms 500a and 500b, an operating portion 600, an input processing circuit 700, an image processing circuit 800, and displays 900a and 900b.
The operating table 100 is a platform on which is placed a patient P who is the subject of observation/ treatment. Multiple slave arms 200a, 200b, 200c, and 200d are installed in the vicinity of the operating table 100. It would also be acceptable to install the slave arms 200a-200d in the operating table 100.
The slave arms 200a, 200b, 200c, 200d are each configured to have a plurality of multi-degree-of-freedom joints. By bending the respective multi-degree-of-freedom joints, the treatment tools or the like installed to the distal end sides (which are the sides facing the body cavity of the patient P) of the slave arms 200a-200d are positioned relative to the patient P who is placed on the operating table 100. Each multi-degree-of-freedom joint is individually driven by a power unit that is not illustrated in the drawing. As a power unit, one may use, for example, a motor (servomotor) which has a servo mechanism provided with an incremental encoder or decelerator or the like. Operational control of the power unit is conducted by slave control circuit 400.
The slave arms 200a-200d have multiple power units for driving the installed treatment tools 240a-240d (not illustrated in the drawing). With respect to this power unit, as well, one may use, for example, a servomotor. Operational control of the power units is conducted by the slave control circuit 400.
In the case where the power units of the slave arms 200a-200d are driven, the amount of the drive force of the power unit is detected by a position detector. Detection signals from the position detector are inputted to the slave control circuit 400, and the amount of the drive force of the slave arms 200a-200d is detected by the slave control circuit 400 by means of these detection signals.
Surgical-use power transmission adapters (hereinafter simply referred to as "adapter") 220a, 220b, 220c, 220d intervened between the slave arms 200a, 200b, 200c, 200d and the treatment tools 240a, 240b, 240c, 240d, respectively connecting the slave arms 200a, 200b, 200c, 200d and the treatment tools 240a, 240b, 240c, 240d. The adapters 220a-220d each have a linear motion mechanism, and are configured to transmit the power generated in the power unit of corresponding the slave arm to the corresponding treatment tool by linear motion.
The slave control circuit 400 is configured to have, for example, a CPU or memory or the like. The slave control circuit 400 stores a prescribed program for conducting control of the slave arms 200a-200d. The slave control circuit 400 controls the operations of the slave arms 200a-200d or the treatment tools 240a-240d according to control signals sent from the input processing circuit 700. That is, the slave circuit 400 specifies the slave arm (or treatment tool) that is the object of operation by a master arm operated by an operator Op based on control signals from the input processing circuit 700. Next, the slave control circuit 400 computes the amount of the drive force required to cause movement of the specified slave arm or the like according to an amount of operation of the master arm by the operator Op.

The slave control circuit 400 then controls the operations of the slave arm or the like that is the object of operation of the master arm according to the amount of the drive force which is computed. At this time, the slave control circuit 400 inputs drive signals to the corresponding slave arm. In addition, the slave control circuit 400 receives detection signals that are inputted from the position detector of the power unit according to the operations of the corresponding slave arm, and controls the size and polarity of the drive signals so that the amount of the drive force of the slave arm that is the object of operation is an amount of the drive force which is the target.

The master arms 500a and 500b are configured with a plurality of link mechanisms. A position detector such as, for example, an incremental encoder is provided in each link composing a link mechanism. The operations of each link are sensed by this position detector, and the amount of operation of the master arms 500a and 500b are detected by the input processing circuit 700.

The medical manipulator system of Fig. 1 is a system which operates four slave arms using the two master arms 500a and 500b. Accordingly, the need arises to appropriately switch among the slave arms that are the object of operation of the master arms. Such switching is, for example, conducted by operation of the operating portion 600 by the operator Op. It is needless to say that if a one-to-one correspondence is established with respect to objects of operation by having the same number of master arms and slave arms, such switching is not necessary.

The operating portion 600 has various operation members such as a switch button for switching among slave arms that are the object of operation of the master arms 500a and 500b, a scaling modification switch for modifying the movement ratio of master and slave, and a foot switch for emergency stoppage of the system. When any of the operating members configuring the operating portion 600 is conducted by the operator Op, an operating signal corresponding to operation of the corresponding operating member is inputted to the input processing circuit 700 from the operating portion 600.

The input processing circuit 700 analyzes operating signals from the master arms 500a and 500b and operating signals from the operating portion 600. The input processing circuit 700 then generates control signals for controlling this medical manipulator system according to the results of analysis of the operating signals, and inputs the control signals to the slave control circuit 400.

The image processing circuit 800 executes various kinds of image processing to image signals inputted from the slave control circuit 400, and generates image data for display by the operator-use display 900a and the auxiliary display 900b. The operator-use display 900a and the auxiliary display 900b are configured, for example, with a liquid crystal display, and display images based on image data generated by the image processing circuit 800 according to the image signals obtained via viewing devices.

With the medical manipulator system configured as above, when the operator Op operates the master arm 500a or 500b, the slave arm corresponding to the master arm and the treatment tool installed to that slave arm are operated according to the movements of the master arm 500a or 500b. By this means, the desired procedure can be conducted on the patient P.

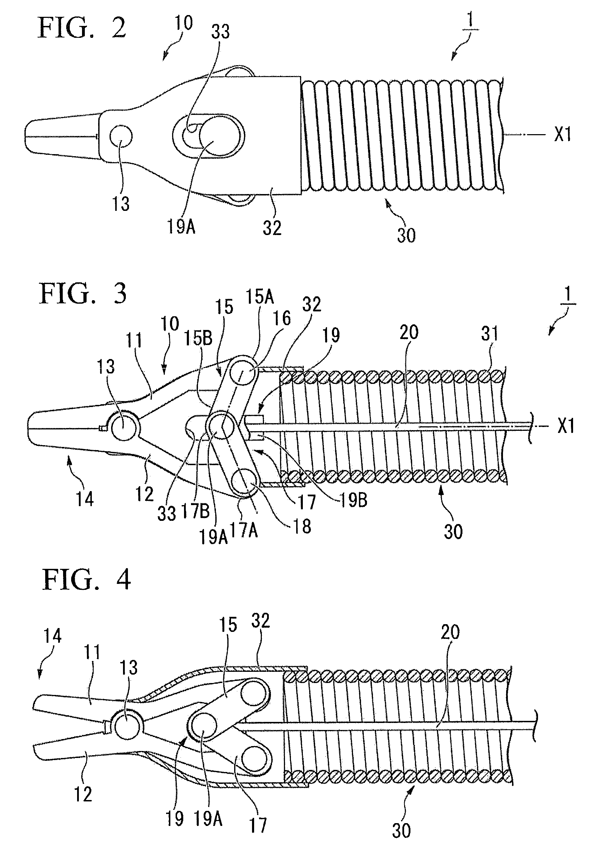

Next, a description is given of a treatment tool 1 of the present embodiment. Fig. 2 is a diagram which shows the distal end side of the treatment tool 1. Fig. 3 is a diagram which shows the interior of the distal end side of the treatment tool 1. The treatment tool 1 may be installed to the slave arms 200a-200d as the treatment tools 240a-240d. As shown in Fig. 2 and Fig. 3, the treatment tool 1 is provided with an end effector 10 for conducting various treatments, an operating member 20 for operating the end effector 10, and a sheath portion 30 which the operating member 20 is inserted into.

The end effector 10 is provided with a pair of forceps pieces (jaw members) composed of a first forceps piece 11 and a second forceps piece 12. The first forceps piece 11 and second forceps piece 12 are rotatably connected to each other by a forceps rotating shaft 13. In the first forceps piece 11 and second forceps piece 12, the region which is further toward the distal end side than the forceps rotating shaft 13 configures a gripping portion 14 that opens and closes, and that grips objects such as tissue in the body and surgical instruments.

At the proximal end side of the first forceps piece 11 which is the opposite side of the gripping portion 14, a distal end portion (first end portion) 15A of a link member 15 is rotatably coupled to the first forceps piece 11 via a link rotating shaft 16. Similarly, at the proximal end side of the second forceps piece 12, a distal end portion (first end portion) 17A of a link member 17 is rotatably coupled to the second forceps piece 12 via a link rotating shaft 18. The axes line of the link rotating shafts 16 and 18 are respectively parallel to the axis line of the forceps rotating shaft 13. Moreover, the respective distal end portions 15A and 17A of the respective link members 15 and 17 are connected further toward the operating member 20 side than the forceps rotating shaft 13.

The proximal end portions (second end portions) 15B and 17B of the link members 15 and 17 are rotatably connected to a connecting member 19 via a connecting shaft 19A. The axis line of the connecting shaft 19A is parallel to the forceps rotating shaft 13 and the link rotating shafts 16 and 18, and each link member 15 and 17 is capable of relative rotation with respect to the connecting member 19.

The connecting member 19 is formed with metal or the like, and has a connecting shaft 19A on its distal end side. The proximal end side of the connecting member 19 is an operation member connection 19B formed in an approximately cylindrical shape. The distal end portion of the operating member 20 is inserted into the operation member connection 19B, and is integrally connected by welding or adhesion, or by swaging or the like.

The operating member 20 is an elongate member, and has rigidity such that it is possible to transmit advance or retract movement operation at its proximal end portion to its distal end portion. The operating member 20 is integrally connected with the connecting member 19 on the proximal end side of the end effector 10 by insertion of its distal end portion into the operating member connection 19B of the connecting member 19.

The sheath portion 30 is provided with a sheath 31 that is cylindrically formed. The operating member 20 is inserted into the sheath portion 30 in a manner enabling advancing or retracting movement. In the present embodiment, a conventional flexible coil sheath is used as the sheath 31.

As shown in Fig. 2, a cover member (base) 32 made from metal or the like is attached to the distal end portion of the sheath 31. The forceps rotating shaft 13 is fixed to the cover member 32. That is, the first forceps piece 11 and the second forceps piece 12 are supported so as to be capable of rotating relative to the cover member 32, and the forceps rotating shaft 13 is fixed so that it does not move relative to the sheath portion 30.

Moreover, as shown in Fig. 2, an elongate hole (stopper portion) 33 which extends along the axis line X1 of the operating member 20 is formed in the cover member 32. The connecting shaft 19A is inserted into the elongate hole 33. When the operating member 20 advances or retracts along axis line X1, the proximal end portions 15B and 17B of the respective link members connected to the connecting shaft 19A are moved along the elongate hole 33. Below, the straight line constituting the trajectory of the connecting shaft 19A during advance or retract movement of the operating member 20 is referred to as "the drive axis of the proximal end portion".

A description will now be given of an example of operations during use of the treatment tool 1 configured in the foregoing manner, for the case where it is installed to one of the slave arms 200a-200d.

First, the operator Op installs the treatment tool 1 to the desired slave arm, and connects the proximal end portion of the operating member 20 to the adapter of the slave arm.

When the operator Op operates the prescribed operation to the corresponding master arm, the power unit of the slave arm is driven via the slave control circuit 400. The power generated by the power unit is converted to linear motion by the adapter, and the operating member 20 is advanced or retracted along axis line X1 by linear motion.

When the operating member 20 is advanced, the connecting member 19 which is connected to the operating member 20 advances relative to the sheath portion 30. However, as the forceps rotating shaft 13 is fixed to the cover member 32, it does not advance relative to the sheath portion 30. By this means, the connecting shaft 19A approaches the forceps rotating shaft 13, and the link members 15 and 17 rotate relative to the respective forceps pieces 11 and 12 and the connecting member 19. As a result, the first forceps piece 11 and the second forceps piece 12 are rotated centering on the forceps rotating shaft 13, and the gripping portion 14 is opened as shown in Fig. 4.

When the operating member 20 is retracted, the connecting shaft 19A moves away from the forceps rotating shaft 13, and the gripping portion 14 is closed by a movement that is the reverse of the above-described movement. Accordingly, by moving the operating member 20 advance or retract relative to the cover member 32 along axis line X1, the gripping portion 14 is opened or closed, and the desired procedure such that gripping subject tissue, or gripping the implement required for treatment such as a curved needle or suture thread can be conducted.

Fig. 3 shows the condition where the first forceps piece 11 and the second forceps piece 12 are in contact further toward the distal end side than the forceps rotating shaft 13, and the gripping portion 14 is closed. In the condition where this gripping portion 14 is closed, the connecting shaft 19A is positioned further toward the distal end side than the respective link rotating shafts 16 and 18. That is, the proximal end portions 15B and 17B of the respective link members 15 and 17 are positioned further toward the distal end side than the distal end portions 15A and 17A.

Moreover, the distance between the link rotating shaft 16 and the drive axis of the proximal end portions 15B and 17B of the respective link members 15 and 17 is shorter than the length of the link member 15. Similarly, the distance between the link rotating shaft 18 and the drive axis of the proximal end portions is shorter than the length of the link member 17.

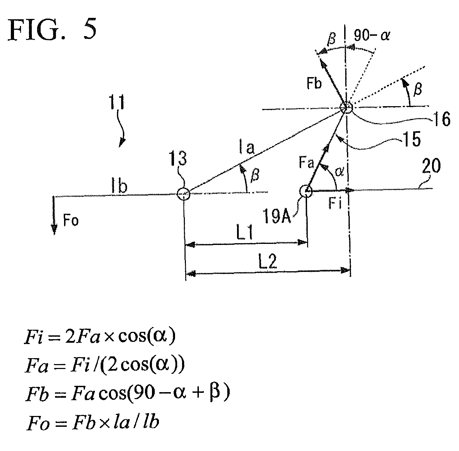

As shown in Fig. 5, the length of projection along the drive axis of the proximal end portions of a line segment which connects the center of rotation of the pair of jaw members and the proximal end portions of the link members is considered as a length L1. Moreover, the length of projection on the drive axis of the proximal end portions of a line segment which connects the center of rotation and the distal end portions of the link members is considered as a length L2. This length L1 is set to be shorter than the length L2.

This is now described with respect to the present embodiment. In the present embodiment, the center of rotation of the pair of jaw members is the central axis of the forceps rotating shaft 13. The proximal end portions of the link members have approximately the same position as the position of the connecting axis 19A. Furthermore, the distal end portions of the link members have approximately the same position as the link rotating shaft 16. Accordingly, the length L1 is the length of projection of a line segment which connects the proximal end portions on the drive axis of the proximal end portions of the link members and the central axis of the forceps rotating shaft. In the present embodiment, this length L1 is set so as to be shorter than the length L2. The details of Fig. 5 are described below.

According to this type of configuration, the connecting member 19, the respective link members 15 and 17, and the respective forceps pieces 11 and 12 function as a so-called toggle mechanism. Accordingly, from a condition where the gripping portion 14 is closed, the respective link members 15 and 17 and the respective forceps pieces 11 and 12 are elastically deformed by further conducting operating input with respect to the operating member 20 in the direction of retraction of the operating member 20. The proximal end portions 15B and 17B of the respective link members connected to the connecting member 19 are then moved along the axis line X1 of the operating member 20. That is, the drive axis of the proximal end portions is parallel (including approximately parallel) with the axis line X1. At this time, the external appearance of the closed gripping portion 14 hardly changes, but the gripping force generated by the gripping portion 14 intensifies.

The details of the aforementioned intensification of gripping force are described. Fig. 5 schematically illustrates the first forceps piece 11, the forceps rotating shaft 13, the link member 15, the link rotating shaft 16, the connecting shaft 19A, and the operating member 20. As shown in Fig. 5, when a manipulation input Fi acts upon the operating member 20 to retract the operating member 20, the connecting shaft 19A retracts. And then, an angle $\alpha$ constituted by the link member 15 and the drive axis of the proximal end portion on the proximal end side increases, and a force Fb which moves the link rotating shaft 16 in a direction that distances it from the axis line of the operating member 20 is generated. The force Fb acts as a force that rotates the respective forceps pieces 11 and 12 around the forceps rotating shaft 13, ultimately generating an output Fo in the gripping portion 14. The reference code 1a shown in Fig. 5 indicates the length of the region of the respective forceps piece on the proximal end side from the forceps rotating shaft 13. The reference code 1b indicates the length of the region of the respective forceps piece on the distal end side from the forceps rotating shaft 13. An angle $\beta$ refers to an angle constituted by the drive axis of the connecting shaft 19A and a line segment which connects the forceps rotating shaft 13 and the link rotating shaft 16. Although the second forceps piece 12 and the like are not illustrated, a similar output Fo is generated with these.

In the respective forceps pieces 11 and 12, the size of the output Fo generated in the gripping portion 14 is expressed by the following Formula 1.
(Formula 1)

$$Fo = \frac{Fi\cos(90 - \alpha + \beta)\, la}{2\cos(\alpha)\, lb}$$

Accordingly, as angle $\alpha$ approaches 90 degrees, the output Fo grows exponentially increases. Theoretically, the output Fo could be made infinite. However, in reality, when the output Fo reaches a prescribed level or higher, the respective forceps pieces 11 and 12 and the respective link members 15 and 17 undergo plastic deformation, with the result that the upper limit of gripping force is prescribed by the yield stress of these members.

The form of the elongate hole 33 formed in the cover member 32 and its dimensions in the direction of movement of the operating member 20 are set taking into consideration the form of the object to be gripped and the yield stress. Consequently, under conditions where an object is gripped, even when the operating member 20 is retracted until the connecting shaft 19A contacts the proximal end of the elongate hole 33, the respective forceps pieces 11 and 12 and the respective link members 15 and 17 do not undergo plastic deformation. That is, the elongate hole 33 functions as a guide which causes movement of the proximal end portion of each link member that is joined to the connecting shaft 19A along the axis line X1 of the operating member 20. Moreover, the elongate hole 33 also functions as a stopper which regulates the maximal retraction amount of the operating member 20 by contact with the connection/rotating shaft 19A.

As described above, according to the treatment tool 1 of the present embodiment, a toggle mechanism is configured by the respective forceps pieces 11 and 12, the respective link members 15 and 17, and the connecting member 19 of the end effector 10. Consequently, with the treatment tool 1 of the present embodiment, it is possible to efficiently intensify the gripping force generated in the gripping portion 14 even with a comparatively small manipulation input by further conducting a manipulation input that retracts the operating member 20 from the state where the gripping portion 14 is closed.

Moreover, the respective link members 15 and 17 are connected to the respective forceps pieces 11 and 12 and the operating member 20 by the link rotating shafts 16 and 18 and the connecting shaft 19A, and are not connected via an elongate hole like that described in Patent Document 2. Consequently, the respective link members 15 and 17 do not slide against other members when gripping force intensifies. As a result, there is no occurrence of friction due to sliding, manipulation inputs can be efficiently transmitted to the end effector, and gripping force can be efficiently intensified.

Here, the reactive force of the output Fo acts upon the connecting shaft 19A upon which the manipulation input Fi acts so that it is distanced from the axis line of the operating member 20. However, as the connecting shaft 19A is positioned at approximately midway between the link rotating shafts 16 and 18 in the opening/closing direction of the gripping portion 14, the reactive force of the output Fo exerted upon the respective link rotating shafts is exerted in roughly the opposite direction. As a result, the input Fi and the output Fo offset, and become approximately zero. Accordingly, the connecting shaft 19A that inserted into the elongate hole 33 does not strongly press against the inner surface of the elongate hole 33, inhibiting occurrence of excessive friction.

Furthermore, as mentioned above, the elongate hole 33 also functions as a stopper. By this means, suitable use can be conducted, because it is possible to prevent breakage of the toggle mechanism due to the aforementioned length L1 equaling or exceeding to the length L2 as a result of plastic deformation of the forceps pieces and the link members, movement of the connection/rotating shaft 19A further toward the proximal end side than the link rotating shafts 16 and 18.

With respect to the present embodiment, the case was described where the stopper is an elongate hole, but instead of this, it is also acceptable to provide a groove or recess that does not pierce the cover member as the stopper, and to have a configuration where the connection/rotating shaft 19A is inserted into the groove.

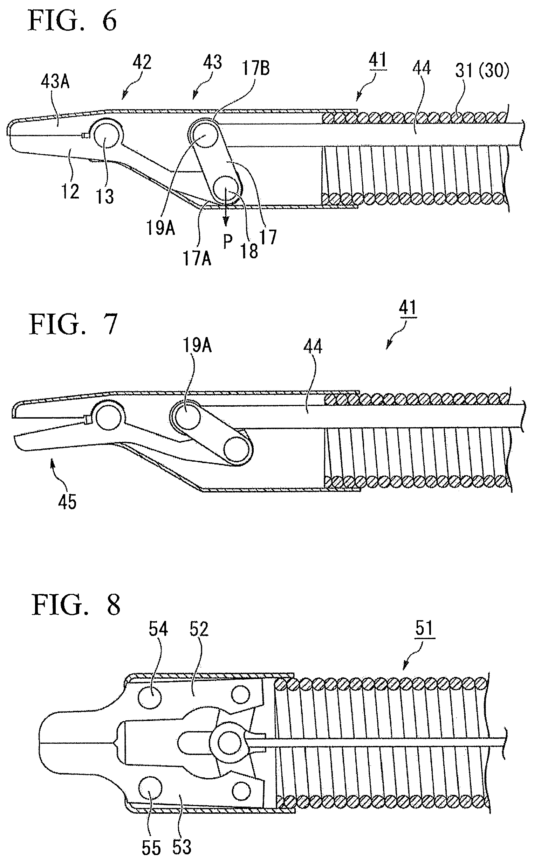

Next, a second embodiment of the present invention is described with reference to Fig. 6 and Fig. 7. A treatment tool 41 of the present embodiment differs from the above-described treatment tool 1 in that only one forceps piece of the pair of forceps pieces is capable of rotation. In the following description, components with the same configuration as those already described are assigned the same reference numerals, and duplicative description thereof is omitted.

Fig. 6 is a diagram which shows an end effector portion 42 of the treatment tool 41, and its periphery. The treatment tool 41 is provided with a cover member 43 having a forceps piece portion 43A corresponding to a first forceps piece among a pair of forceps pieces. In the treatment tool 41, only the second forceps piece 12 is configured to be capable of rotation around the forceps rotating shaft 13. Consequently, the treatment tool 41 is not provided with the link member 15 and the link rotating shaft 16.

An operating member 44 is a rod having a prescribed rigidity. The distal end portion of the operating member 44 is connected to the link member 17 via the connecting shaft 19A. The positional relations of the distal end portion 17A and the proximal end portion 17B of the link member 17 in a state where the forceps piece portion 43A and the second forceps piece 12 are closed by contact, the connection length of the link member, and the like are set to be approximately identical to those of the treatment tool 1.

The cover member 43 is not provided with the elongate hole 33. Instead, a stopper, which is not illustrated in the drawings and which controls the amount of retraction of the operating member 44 within prescribed values, is provided at the proximal end side of the operating member 44. The maximal amount of retraction of the operating member 44 is set to a value where the second forceps piece 12 and the link member 17 do not undergo plastic deformation, and where the connecting shaft 19A does not move further toward the proximal end side than the link rotating shaft 18.

With respect to the treatment tool 41 configured as above, such as with the treatment tool 1, when the operating member 44 is advanced, a gripping portion 45 is opened as shown in Fig. 7. After the gripping portion 45 is closed, the gripping force generated in the gripping portion 45 can be intensified by conducting a manipulation input that the operating member 44 is further retracted.

Here, the reactive force of the output Fo that acts upon the link rotating shaft 18 acts upon the operating member 44 so that the connecting shaft 19A is moved away from the axial direction of the operating member 44. The prescribed rigidity of the operating member 44 is set to a level where there is no deformation relative to a maximal reactive force within a range of manipulation of the operating member 44. Accordingly, even when the gripping force is intensified, the connecting shaft 19A resists the reactive force, and moves along the axis line of the operating member 44.

As with the treatment tool 1, with respect also to the treatment tool 41 of the present embodiment, the gripping force produced in the gripping portion can be efficiently intensified even with a comparatively small manipulation input by conducting manipulation input that retracts the operating member.

Moreover, by using the operating member 44 having sufficient rigidity, it is possible to have the connecting shaft 19A move along the axis line of the operating member 44, and to have the toggle mechanism function appropriately, even without providing a guide mechanism such as the elongate hole 33. Furthermore, by using the cover member 43 that has the forceps piece portion 43A, the number of parts can be reduced, and the structure can be simplified.

Descriptions of the respective embodiments of the present invention have been given above, but the technical scope of the present invention is not limited to the foregoing embodiments. Additions, omissions, substitutions, and other modifications can be made without departing from the spirit or scope of the present invention, and the configurations of the respective embodiments may be combined.

In the foregoing embodiments, the description concerned the case where a pair of jaw members is supported by a common rotating shaft. Otherwise, as a third embodiment it is also acceptable to have a pair of forceps pieces 52 and 53 supported by respectively different rotating shafts 54 and 55, as in the treatment tool 51 shown in Fig. 8. By separating the center of rotation of the pair of jaw members, the angle β shown in Fig. 5 can be reduced, and the output Fo generated in the gripping portion can be enhanced.

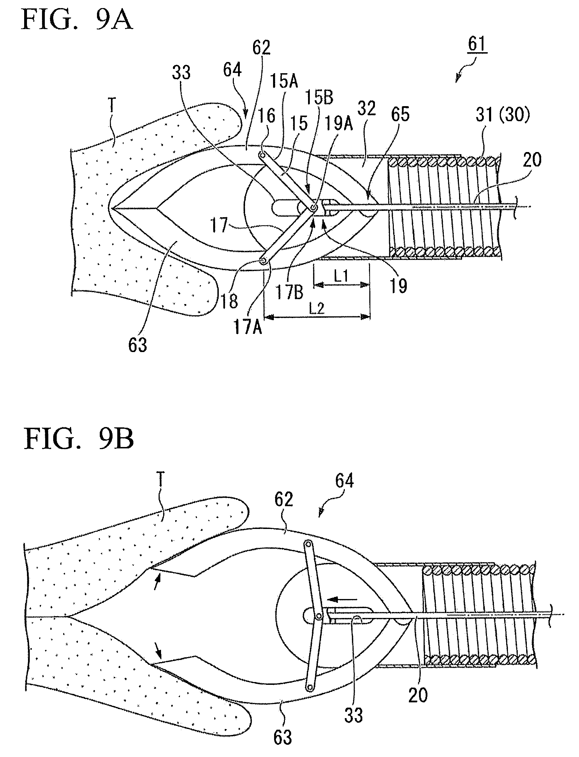

The end effector of the treatment tools is not limited to intensifying force in the direction of closure of the pair of jaw members. As a fourth embodiment, Fig. 9A and Fig. 9B show an embodiment where force is intensified in the direction of opening of the pair of jaw members in the end effector.

A treatment tool 61 shown in Fig. 9A is provided with an end effector 64 having a pair of jaw members 62 and 63. A rotating shaft 65 which constitutes the center of rotation of the pair of jaw members is positioned further toward the proximal end side than the connecting shaft 19A to which the proximal end portions 15B and 17B of the respective link members 15 and 17 are connected. Moreover, the distal end portions 15A and 17A of the respective link members 15 and 17 are positioned further toward the distal end side  (the opposite side of the operating member 20) of the pair of jaw members 62 and 63 than the rotating shaft 65, and the length L1 based on the foregoing definition is set to be shorter than the length L2.

As shown in Fig. 9A, when using the treatment tool 61, the end effector 64 is inserted into an interstice or the like of biological tissue T, and the operating member 20 is advanced, whereupon, as shown in Fig. 9B, the pair of jaw members 62 and 63 is opened, and the biological tissue T can be pushed apart. Within a dimension where the length L1 does not exceed the length L2, the force generated in the end effector 64 can be intensified as the operating member 20 is advanced. By this means, it is possible to have a treatment tool which enables appropriate holding of the biological tissue T in a flared state with resisting the force of the biological tissue T to return to its original state.

Given that such a treatment tool intensifies force by advancement of the operating member, it is preferable to use a member which inhibits deformation relative to the force that causes compression in the axial direction (which has high compressive resistance) as the operating member.

The present embodiment may also be applied in the case where, for example, only one forceps piece of the pair of forceps pieces (jaw members) is capable of rotation, as shown in Fig. 6. It may also be applied in the case where, for example, the pair of forceps  pieces (jaw members) is supported by respectively different rotating shafts, as shown in Fig. 8.

Moreover, a stopper is not indispensable to the treatment tool of each embodiment. Accordingly, it is also acceptable not to provide a stopper, and to use while preventing breakage of the toggle mechanism by having the operator appropriately regulate the amount of retraction movement of the operating member.

In this case, instead of an elongate hole, a groove or a notch which extends in the axial direction of the operating member may be formed in the cover member so as to have only the function of guide of connection/rotating shaft without a stopper function.

However, by providing a stopper, there is the advantage that it is possible to use while preventing breakage of the toggle mechanism without awareness of the foregoing, thereby facilitating manipulation.

[Industrial Applicability]

According to the invention, it is provided that the medical treatment tool and manipulator which are possible to efficiently transmit a manipulation input and to generate a large gripping force with a small manipulation input.

[Description of Reference Numerals]

| | |
|---|---|
| 1, 41, 51, 61 | medical treatment tool |
| 10, 42, 64 | end effector |
| 11 | first forceps piece (jaw members) |
| 12 | second forceps piece (jaw members) |
| 15, 17 | link member |
| 15A, 17A | distal end portion (first end portion) |
| 15B, 17B | proximal end portion (second end portion) |
| 19A | connecting shaft |
| 20, 44 | operating member |
| 32, 43 | cover member (base) |
| 33 | elongate hole (stopper portion) |
| 52, 53 | forceps piece (jaw members) |
| 62, 63 | jaw members |

**Claims**

1.　A medical treatment tool, comprising:

an end effector which has a pair of jaw member of which at least one is supported so that it is capable of being rotated relative to a base;

an operating member which is configured to advance or retract in its own axial direction relative to the base so that the pair of jaw members rotates and opens/closes; and

a link member of which a first end portion of a link member is rotatably coupled to the jaw member, and a second end portion of the link member is coupled to the operating member; wherein

a drive axis of the second end portion is parallel with an axis of the operating member;

a distance between the first end portion and the drive axis of the second end portion is shorter than the length of the link member; and

a length of projection of a line segment which connects the second end portion and a center of rotation of the pair of jaw members on the drive axis is shorter than a length of projection of a line segment which connects to the first end portion and the center of rotation on the drive axis.

2. The medical treatment tool according to claim 1, wherein

the first end portion of the link member is connected to the operating member on the operating member side from the center of rotation of the pair of jaw members.

3. The medical treatment tool according to claims 1 or 2, further comprising

a stopper being configured to prevent the length of projection of the line segment which connects the second end portion and the center of rotation on the drive axis from being equal to or more than the length of projection of the line segment which connects the first end portion and the center of rotation on the drive axis when the operating member is moved.

4. The medical treatment tool according to claim 3, wherein

the stopper is an elongate hole which is formed in the base and which extends in a direction of movement of the operating member, and a connecting shaft which connects the second end portion and the operating member is inserted into the elongate hole.

5. A manipulator equipped with the medical treatment tool according to any one of claims 1 to 4.

FIG. 1

FIG. 2

FIG. 3

FIG. 4

FIG. 5

$$Fi = 2Fa \times \cos(\alpha)$$
$$Fa = Fi / (2\cos(\alpha))$$
$$Fb = Fa\cos(90 - \alpha + \beta)$$
$$Fo = Fb \times la / lb$$

EP 2 666 429 A1

FIG. 6

FIG. 7

FIG. 8

FIG. 9A

FIG. 9B

## INTERNATIONAL SEARCH REPORT

| International application No. |
|---|
| PCT/JP2012/056156 |

A.  CLASSIFICATION OF SUBJECT MATTER
*A61B19/00*(2006.01)i, *A61B17/28*(2006.01)i, *B25J15/08*(2006.01)i

According to International Patent Classification (IPC) or to both national classification and IPC

B.  FIELDS SEARCHED

Minimum documentation searched (classification system followed by classification symbols)
A61B19/00, A61B17/28, B25J15/08

Documentation searched other than minimum documentation to the extent that such documents are included in the fields searched
| | | | |
|---|---|---|---|
| Jitsuyo Shinan Koho | 1922-1996 | Jitsuyo Shinan Toroku Koho | 1996-2012 |
| Kokai Jitsuyo Shinan Koho | 1971-2012 | Toroku Jitsuyo Shinan Koho | 1994-2012 |

Electronic data base consulted during the international search (name of data base and, where practicable, search terms used)

C.  DOCUMENTS CONSIDERED TO BE RELEVANT

| Category* | Citation of document, with indication, where appropriate, of the relevant passages | Relevant to claim No. |
|---|---|---|
| X<br>Y | JP 9-276283 A  (Olympus Optical Co., Ltd.),<br>28 October 1997 (28.10.1997),<br>paragraphs [0085] to [0088]; fig. 19<br>(Family: none) | 1,2<br>3-5 |
| Y | CD-ROM of the specification and drawings annexed to the request of Japanese Utility Model Application No. 47178/1991(Laid-open No. 115/1993)<br>(Olympus Optical Co., Ltd.),<br>08 January 1993 (08.01.1993),<br>paragraphs [0024] to [0025]; fig. 1<br>(Family: none) | 3-5 |

☒  Further documents are listed in the continuation of Box C.          ☐  See patent family annex.

| | |
|---|---|
| *     Special categories of cited documents:<br>"A"   document defining the general state of the art which is not considered to be of particular relevance<br>"E"   earlier application or patent but published on or after the international filing date<br>"L"   document which may throw doubts on priority claim(s) or which is cited to establish the publication date of another citation or other special reason (as specified)<br>"O"   document referring to an oral disclosure, use, exhibition or other means<br>"P"   document published prior to the international filing date but later than the priority date claimed | "T"   later document published after the international filing date or priority date and not in conflict with the application but cited to understand the principle or theory underlying the invention<br>"X"   document of particular relevance; the claimed invention cannot be considered novel or cannot be considered to involve an inventive step when the document is taken alone<br>"Y"   document of particular relevance; the claimed invention cannot be considered to involve an inventive step when the document is combined with one or more other such documents, such combination being obvious to a person skilled in the art<br>"&"   document member of the same patent family |

| Date of the actual completion of the international search | Date of mailing of the international search report |
|---|---|
| 22 March, 2012 (22.03.12) | 03 April, 2012 (03.04.12) |

| Name and mailing address of the ISA/ | Authorized officer |
|---|---|
| Japanese Patent Office | |
| Facsimile No. | Telephone No. |

Form PCT/ISA/210 (second sheet) (July 2009)

## INTERNATIONAL SEARCH REPORT

| | International application No. |
|---|---|
| | PCT/JP2012/056156 |

C (Continuation).   DOCUMENTS CONSIDERED TO BE RELEVANT

| Category* | Citation of document, with indication, where appropriate, of the relevant passages | Relevant to claim No. |
|---|---|---|
| A | JP 2005-237574 A  (Olympus Corp.),<br>08 September 2005 (08.09.2005),<br>fig. 3 to 9<br>& US 2005/0187547 A1     & EP 1568330 A1 | 1-5 |

Form PCT/ISA/210 (continuation of second sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2011054430 A
- JP H61696 A
- JP 2007301692 A